(19) Europäisches Patentamt
European Patent Office
Office européen des brevets

(11) **EP 1 415 150 B1**

(12) **FASCICULE DE BREVET EUROPEEN**

(45) Date de publication et mention
de la délivrance du brevet:
**23.03.2011 Bulletin 2011/12**

(51) Int Cl.:
***G01N 33/24*** *(2006.01)*     ***G01N 33/28*** *(2006.01)*
***E21B 47/10*** *(2006.01)*

(21) Numéro de dépôt: **02764992.0**

(22) Date de dépôt: **24.07.2002**

(86) Numéro de dépôt international:
**PCT/FR2002/002643**

(87) Numéro de publication internationale:
**WO 2003/010534 (06.02.2003 Gazette 2003/06)**

(54) **METHODE DE SUIVI QUANTITATIF D'UN GAZ INJECTE DANS UN RESERVOIR NOTAMMENT DANS UN MILIEU NATUREL**

MESSMETHODE ZUR QUANTITATIVEN BESTIMMUNG EINES EINSPRITZGASES IN EINEM RESERVOIR, INSBESONDERE IN EINER NATÜRLICHEN UMGEBUNG

METHOD FOR QUANTITATIVE MONITORING OF A GAS INJECTED INTO A RESERVOIR IN PARTICULAR IN A NATURAL ENVIRONMENT

(84) Etats contractants désignés:
**CH DE GB LI SE SK TR**

(30) Priorité: **26.07.2001 FR 0110050**

(43) Date de publication de la demande:
**06.05.2004 Bulletin 2004/19**

(73) Titulaire: **Institut Français du Pétrole**
**92852 Rueil-Malmaison Cedex (FR)**

(72) Inventeurs:
• **PRINZHOFER, Alain**
**F-75011 Paris (FR)**
• **ROJEY, Alexandre**
**F-92500 Rueil-Malmaison (FR)**

(56) Documents cités:
**EP-A- 0 816 631    DE-A- 4 323 283**
**US-A- 4 928 522    US-A- 4 972 704**
**US-A- 5 881 807    US-A- 6 035 701**
**US-B1- 6 234 004**

## Description

La présente invention concerne une méthode de suivi quantitatif d'un gaz injecté dans un réservoir tel qu'un milieu naturel.

La méthode trouve des applications dans de nombreux domaines où le gaz injecté dans le réservoir est susceptible de réagir chimiquement avec le milieu d'injection.

Elle permet par exemple de quantifier la fraction d'un gaz injecté dans un réservoir souterrain qui aura réagi chimiquement avec l'encaissant par rapport à la fraction de gaz qui sera restée sous la même forme chimique. Lors du stockage de gaz hydrocarbures naturels pour des raisons de souplesse de distribution, la récupération de ce gaz n'est jamais parfaite. Une partie de ce gaz (principalement du méthane) peut soit rester séquestrée dans certains compartiments du réservoir, soit réagir sous une action chimique ou bactérienne et se transformer en une autre espèce gazeuse ou en solide,

Un autre domaine d'application est par exemple la séquestration du dioxyde de carbone dans des réservoirs souterrains : galeries de mines, bancs de charbon ou anciens réservoirs pétroliers. Il est souhaité qu'une partie de ce $CO_2$ puisse se transformer en solide (carbonates par exemple), afin d'éliminer définitivement le risque de le voir participer à l'effet de serre par échappement dans l'atmosphère. La méthode proposée permet de quantifier la proportion de $CO_2$ transformé en solide au cours du temps, dans la mesure où il est possible de prélever des échantillons de gaz injectés après complétion de l'injection.

## La méthode selon l'invention

La méthode consiste essentiellement à injecter dans le réservoir un mélange du gaz potentiellement réactif à quantifier avec une proportion relativement faible d'un gaz traceur dont l'inertie chimique est totale et à déterminer la variation au cours du temps de la proportion initiale du gaz réactif qui a pu disparaître par transformation, par mesure de la variation de concentration du gaz traceur dans le mélange.

Comme gaz traceur, on peut utiliser par exemple des gaz rares choisis pour être à la fois exempts de problème de contamination, et pour avoir des propriétés physiques telles que la solubilité dans l'eau ou les coefficients de diffusion, les plus proches possibles du gaz injecté.

Comme gaz traceur, on peut utiliser par exemple un isotope de gaz rare choisi pour être à la fois non contaminable et pour avoir des propriétés physiques (telles que la solubilité dans l'eau ou les coefficients de diffusion), les plus proches possibles du gaz injecté, la détermination de la variation au cours du temps de la proportion initiale du gaz réactif qui a pu disparaître par transformation comportant une mesure de concentration par dilution isotopique.

La méthode comporte par exemple l'injection dans le réservoir d'un mélange de méthane et d'un gaz rare tel que l'argon.

La méthode peut comporter également par exemple l'injection dans le réservoir d'un mélange de dioxyde de carbone et d'un gaz rare tel que le xénon ou le krypton.

La mesure de la variation au cours du temps de la proportion initiale du gaz réactif peut être effectuée par chromatographie en phase gazeuse, par spectrométrie quadrupôlaire ou par couplage d'une ligne de préparation à un spectromètre à secteur magnétique.

## Présentation sommaire des figures

D'autres caractéristiques et avantages de la méthode selon l'invention, apparaîtront à la lecture de la description ci-après, en se référant aux dessins annexés où :

- la Fig.1 est un tableau des valeurs de solubilité pour deux espèces réactives : méthane et dioxyde de carbone, et pour les espèces de la famille des gaz rares (He, Ne, Ar, Kr, Xe) ;

- la Fig.2 montre les valeurs de solubilité (exprimées comme inverses de la constante de Henry en $atm^{-1}$ pour les deux espèces réactives ($CH_4$, $CO_2$) et pour la même famille de gaz rares ;

- la Fig.3 montre sous forme graphique, les valeurs (en $cm^2 s^{-1}$) des coefficients de diffusion dans l'eau pour les deux espèces réactives ($CH_4$, $CO_2$), ainsi que pour les espèces de la même famille de gaz rares ; et

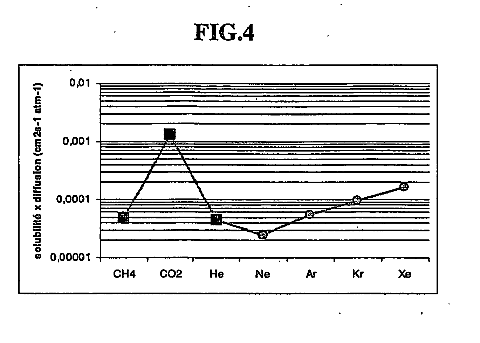

- la Fig.4 montre sous forme graphique, le produit de la solubilité par le coefficient de diffusion, dans les mêmes unités que pour les Fig.1, 2, pour les mêmes espèces réactives et espèces de la même famille de gaz rares.

## DESCRIPTION DETAILLEE

La méthode comporte comme on l'a vu, l'injection dans un réservoir tel qu'un réservoir souterrain, d'un mélange du gaz potentiellement réactif à quantifier avec une proportion relativement faible d'une autre espèce (un gaz traceur) dont l'inertie chimique est totale et à mesurer la variation au cours du temps de la proportion initiale du gaz réactif qui a pu disparaître par transformation.

Les deux processus physiques envisageables affectant l'espèce réactif et le traceur gaz rare sont les phénomènes de solubilisation du gaz dans l'eau, et la diffusion des espèces gazeuses, moléculaires dans l'eau. En effet, la diffusion dans la phase gaz peut être considérée comme négligeable au vu des temps envisageables entre chaque prélèvement (mois ou années). Un autre facteur important pour ces phénomènes de transport dissolution est le produit de la solubilité par la diffusion, comme décrit par exemple par :

- Krooss B.M. (1992): Diffusive Loss of Hydrocarbons through Cap Rocks. Erdöl & Kohle - Erdgas - Petrochemie/ hydrocarbon Technology, 45, p. 387-396.

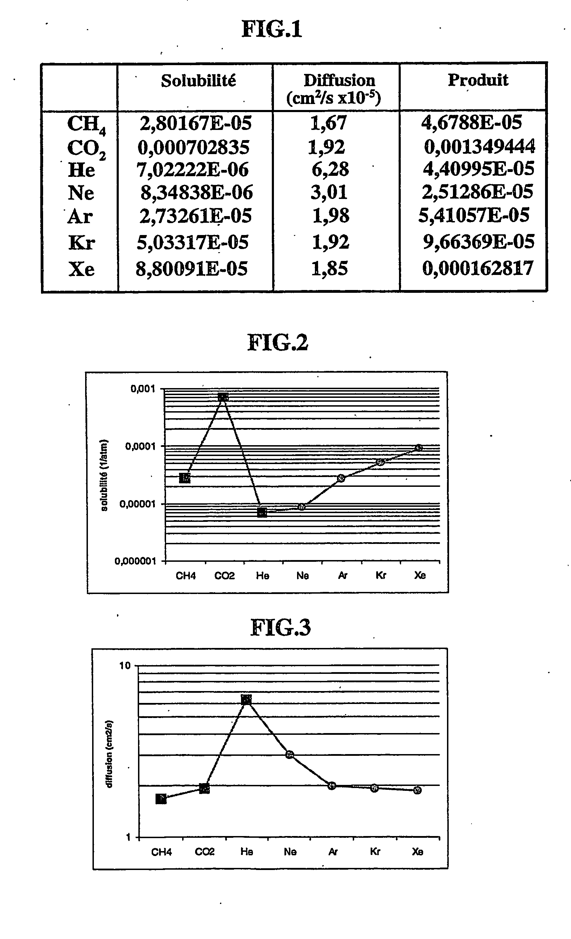

Le tableau de la Fig.1, on le rappelle, donne pour les deux espèces réactifs considérées (méthane et dioxyde de carbone) et pour la famille des gaz rares, les valeurs des coefficients de solubilité dans l'eau douce à 20°C et 1 atmosphère de gaz, les coefficients de diffusion de ces espèces dans l'eau, ainsi que le produit de ces deux paramètres. Les valeurs de ces paramètres seront différentes pour d'autres températures, pressions et composition de la phase aqueuse, mais en première approximation, ces variations seront du même ordre pour toutes les espèces considérées, et les rapports entre espèces, qui seuls nous intéressent, resteront quasiment identiques.

Il apparaît que le gaz rare dont les propriétés physiques se rapprochent le plus de celles du méthane, est l'argon. La grande solubilité du $CO_2$ dans l'eau ne correspond à aucun gaz rare, le xénon étant le gaz qui s'en rapproche le plus. Cependant, le xénon étant un gaz rare qui s'adsorbe facilement sur les solides, nous préconisons l'utilisation du krypton, dont les propriétés restent proches de celles du xénon, et qui ne présente que dans une moindre mesure ce problème de piégeage par adsorption.

Calcul de la quantité à injecter par rapport à la précision des analyses et la précision sur la proportion de gaz injecté ayant réagi chimiquement

La proportion de gaz rare à injecter dans le méthane ou le dioxyde de carbone dépend de la précision analytique envisagée, et des précautions que l'on désire prendre avec la contamination par l'air et les aquifères environnant le lieu de stockage. En effet, les teneurs en argon de l'air sont proches de 1%, les aquifères en sont également très riches, ce qui oblige soit à utiliser un traceur plus concentré (mais des concentrations supérieures à 1% deviennent une gêne pour la qualité du gaz injecté), soit à utiliser un isotope peu abondant de l'argon (36Ar ou 38Ar). Pour le traçage par le Krypton, les teneurs dans l'air sont beaucoup plus faibles (X ppm), et le problème de contamination est moindre. Par contamination, on désigne la modification du taux initial de gaz rare par apport au mélange injecté du même gaz traceur fourni par contact avec le milieu d'injection. L'avantage du traçage par un gaz rare de composition isotopique différente de celle de l'air (ou des milieux naturels concernés par le stockage) est à la fois de s'affranchir de problème de contamination, mais également permet d'effectuer une mesure par dilution isotopique, mesures de concentrations les plus précises à ce jour. La méthode d'analyse peut être, en augmentant la sensibilité et la précision du résultat, la chromatographie en phase gazeuse (uniquement sensible aux espèces chimiques, sans distinction d'isotopes), la spectrométrie quadrupôlaire (même limitation mais sensibilité plus grande) ou le couplage d'une ligne de préparation à un spectromètre à secteur magnétique, qui permet une mesure précise des concentrations et des rapports isotopiques. La quantité de gaz ayant réagi chimiquement après injection peut être calculée suivant la formule :

$$Q = T \, (R - R_o)$$

Avec Q= quantité de gaz ayant réagi, T = quantité de traceur injecté, $R_o$ : rapport entre la quantité de gaz et de traceur au moment de l'injection, R : rapport entre la quantité de gaz résiduel et de traceur au moment d'un prélèvement. On peut estimer que la précision de la mesure d'un rapport R est en routine de quelques pour cent, ce qui donne une précision équivalente sur l'estimation de la quantité de gaz ayant réagi chimiquement. Si la mesure est effectuée par chromatographie, la limite de sensibilité d'un chromatographe normal équipé d'un détecteur TCD est d'environ 0.1%, ce qui obligera donc à charger le dioxyde de carbone à injecter d'un volume de Krypton pour au moins 1000 volumes de dioxyde de carbone. Pour l'argon, nous avons vu que le traçage par un mélange d'isotopes en proportion différente que dans les milieux naturels était indispensable. La méthode d'analyse par chromatographie (comme celle par spectrométrie quadrupôle) est donc inadaptée.

Si on utilise un spectromètre isotopique à secteur magnétique, il est alors possible d'utiliser des traceurs de composition isotopique différente, ce qui à la fois améliore la qualité des mesures et diminue les quantités que l'on doit injecter. Par exemple, un traceur constitué essentiellement de 38Ar se superposerait aux teneurs atmosphériques d'environ 5 $10^{-6}$. Une injection à hauteur de 20 $10^{-6}$ (mole/mole) serait donc parfaitement suffisante. Pour le krypton, le 78 kr par exemple qui a une concentration dans l'atmosphère de 4 $10^{-9}$ pourrait être utilisé à une teneur de $10^{-6}$ permettant une mesure facile, sans même avoir à tenir compte de la pollution par le milieu environnant.

Mise en oeuvre

La méthode peut être utilisée pour tester dans un premier temps un site souterrain en vue de déterminer ses aptitudes pour le stockage de gaz. Dans ce cas, on fore un trou de relativement faible diamètre (« slim hole ») jusqu'à la zone à tester et l'on injecte un mélange homogène de gaz et du gaz traceur, durant un temps qui peut être relativement court de l'ordre de quelques jours à quelques mois. Les prélèvements en vue de mesurer la variation du taux de gaz traceur dans le mélange injecté à l'issue de son temps de séjour, sont faits par ouverture d'une vanne dans le trou d'injection.

La méthode peut également être utilisée pour surveiller l'évolution du contenu d'un gisement de gaz en cours d'exploitation (monitoring) dans le but de détecter des variations de composition d'un mélange qui y est injecté. Par des mesures de la variation du taux de gaz traceur dans du dioxyde de carbone, on peut par exemple suivre sa transformation éventuelle en carbonates au contact du milieu. Le puits par où on effectue les prélèvements, peut être différent de celui par lequel on injecte le gaz.

La fréquence de prélèvements après injection dans le cadre de cette surveillance peut varier entre quelques mois à quelques années.

**Revendications**

1.  Méthode de suivi quantitatif d'un gaz injecté dans un réservoir, susceptible de réagir chimiquement avec le milieu d'injection, **caractérisée en ce qu'**elle comporte l'injection dans le réservoir d'un mélange du gaz avec une proportion faible d'un gaz traceur dont l'inertie chimique est totale, et la détermination de la variation au cours du temps de la proportion initiale du gaz qui a pu disparaître par transformation, par mesure de la variation de concentration du gaz traceur dans le mélange.

2.  Méthode selon la revendication 1, **caractérisé en ce que** l'on utilise comme gaz traceur, un gaz rare choisi non susceptible d'être contaminé par contact du mélange avec le milieu d'injection, et pour avoir des propriétés physiques telles que la solubilité dans l'eau ou les coefficients de diffusion, les plus proches possibles du gaz injecté.

3.  Méthode selon la revendication 1, **caractérisé en ce que** l'on utilise comme gaz traceur, un isotope de gaz rare choisi non susceptible d'être contaminé par contact du mélange avec le milieu d'injection, et pour avoir des propriétés physiques telles que la solubilité dans l'eau ou les coefficients de diffusion, les plus proches possibles du gaz injecté, la détermination de la variation au cours du temps de la proportion initiale du gaz réactif comportant une mesure de concentration par dilution isotopique.

4.  Méthode selon l'une des revendications précédentes, **caractérisée en ce qu'**elle comporte l'injection dans le réservoir d'un mélange de méthane et d'un gaz rare tel que de l'argon.

5.  Méthode selon l'une des revendications précédentes, **caractérisée en ce qu'**elle comporte l'injection dans le réservoir d'un mélange de dioxyde de carbone et d'un gaz rare tel que du xénon ou du krypton.

6.  Méthode selon l'une des revendications précédentes, **caractérisée en ce que** la mesure de la variation au cours du temps de la proportion initiale du gaz réactif est effectuée par chromatographie en phase gazeuse.

7.  Méthode selon l'une des revendications précédentes, **caractérisée en ce que** la mesure de la variation au cours du temps de la proportion initiale du gaz réactif est effectuée par spectrométrie quadrupôlaire.

8.  Méthode selon l'une des revendications précédentes, **caractérisée en ce que** la mesure de la variation au cours du temps de la proportion initiale du gaz réactif est effectuée par couplage d'une ligne de préparation à un spectromètre à secteur magnétique.

**9.** Application de la méthode selon l'une des revendications 1 à 8 à l'évaluation de l'aptitude d'une zone souterraine, au stockage de gaz.

**10.** Application de la méthode selon l'une des revendications 1 à 8 à la surveillance d'un gisement souterrain servant au stockage d'un gaz.

**Claims**

**1.** A method intended for quantitative monitoring of a gas injected into a reservoir, likely to react chemically with the injection medium, **characterised in that** it comprises injecting into the reservoir a mixture of the gas with a low proportion of a tracer gas whose chemical inertia is total, and determining the variation with time of the initial proportion of gas that may have disappeared through conversion, by measuring the concentration variation of the tracer gas in the mixture.

**2.** A method as claimed in claim 1, **characterised in that** the tracer gas used is a rare gas unlikely to be contaminated by contact of the mixture with the injection medium, and which has physical properties such as solubility in water or diffusion coefficients as close as possible to those of the gas injected.

**3.** A method as claimed in claim 1, **characterised in that** the tracer gas used is a rare gas isotope unlikely to be contaminated by contact of the mixture with the injection medium, and which has physical properties such as solubility in water or diffusion coefficients as close as possible to those of the gas injected, determination of the variation with time of the initial proportion of the reactive gas comprising a concentration measurement by isotopic dilution.

**4.** A method as claimed in any one of the previous claims, **characterised in that** it comprises injecting into the reservoir a mixture of methane and of a rare gas such as argon.

**5.** A method as claimed in any one of the previous claims, **characterised in that** it comprises injecting into the reservoir a mixture of carbon dioxide and of a rare gas such as xenon or krypton.

**6.** A method as claimed in any one of the previous claims, **characterised in that** measurement of the variation with time of the initial proportion of reactive gas is carried out by gas chromatography.

**7.** A method as claimed in any one of the previous claims, **characterised in that** measurement of the variation with time of the initial proportion of reactive gas is carried out by quadrupole spectrometry.

**8.** A method as claimed in any one of the previous claims, **characterised in that** measurement of the variation with time of the initial proportion of reactive gas is carried out by coupling a preparation line to a magnetic spectrometer.

**9.** Application of the method as claimed in any one of claims 1 to 8 for evaluation of the gas storage ability of an underground zone.

**10.** Application of the method as claimed in any one of claims 1 to 8 for monitoring of an underground reservoir used for gas storage.

**Patentansprüche**

**1.** Verfahren zur quantitativen Weiterverfolgung eines Gases, das in einen Raum eingeleitet wird und möglicherweise mit dem Einleitungsmilieu chemisch reagiert, **dadurch gekennzeichnet, dass** es das Einleiten einer Mischung des Gases mit einem geringen Anteil eines Spurengases, welches chemisch vollkommen inert ist, in einen Raum aufweist, sowie die Bestimmung der Veränderung, die der ursprüngliche Anteil des Gases im Laufe der Zeit erfährt, indem es durch Umwandlung verschwindet, durch Messen der Veränderung der Konzentration des Spurengases in der Mischung.

**2.** Verfahren nach Anspruch 1, **dadurch gekennzeichnet, dass** als Spurengas ein Edelgas verwendet wird, das derart gewählt wird, dass es durch den Kontakt der Mischung mit dem Einleitungsmilieu nicht kontaminiert werden kann und dass es physikalische Eigenschaften, wie etwa die Wasserlöslichkeit oder die Diffusionskoeffizienten aufweist, welche dem eingeleiteten Gas so nahe wie möglich kommen.

3. Verfahren nach Anspruch 1, **dadurch gekennzeichnet, dass** als Spurengas ein Edelgasisotop verwendet wird, das derart gewählt wird, dass es durch den Kontakt der Mischung mit dem Einleitungsmilieu nicht kontaminiert werden kann und dass es physikalische Eigenschaften, wie etwa die Wasserlöslichkeit oder die Diffusionskoeffizienten aufweist, welche dem eingeleiteten Gas so nahe wie möglich kommen, wobei die Bestimmung der Veränderung, die der ursprüngliche Anteil des reaktiven Gases im Laufe der Zeit erfährt, eine Messung der Konzentration durch Isotopenverdünnung aufweist.

4. Verfahren nach einem der vorhergehenden Ansprüche, **dadurch gekennzeichnet, dass** es das Einleiten einer Mischung aus Methan und einem Edelgas wie etwa Argon in den Raum aufweist.

5. Verfahren nach einem der vorhergehenden Ansprüche, **dadurch gekennzeichnet, dass** es das Einleiten einer Mischung aus Kohlendioxid und einem Edelgas wie etwa Xenon oder Krypton in den Raum aufweist.

6. Verfahren nach einem der vorhergehenden Ansprüche, **dadurch gekennzeichnet, dass** die Messung der Veränderung, die der ursprüngliche Anteil des reaktiven Gases im Laufe der Zeit erfährt, mittels Gaschromatographie durchgeführt wird.

7. Verfahren nach einem der vorhergehenden Ansprüche, **dadurch gekennzeichnet, dass** die Messung der Veränderung, die der ursprüngliche Anteil des reaktiven Gases im Laufe der Zeit erfährt, mittels Quadrupol-Spektrometrie durchgeführt wird.

8. Verfahren nach einem der vorhergehenden Ansprüche, **dadurch gekennzeichnet, dass** die Messung der Veränderung, die der ursprüngliche Anteil des reaktiven Gases im Laufe der Zeit erfährt, mittels Kopplung einer Aufbereitungsleitung an ein magnetisches Sektorfeld-Spektrometer durchgeführt wird.

9. Anwendung des Verfahrens nach einem der Ansprüche 1 bis 8 auf die Bewertung der Eignung eines unterirdischen Bereichs für die Lagerung von Gas.

10. Anwendung des Verfahrens nach einem der Ansprüche 1 bis 8 auf die Überwachung einer unterirdischen Lagerstätte, die zur Lagerung eines Gases dient.

## FIG.1

|  | Solubilité | Diffusion (cm²/s x10⁻⁵) | Produit |
|---|---|---|---|
| CH₄ | 2,80167E-05 | 1,67 | 4,6788E-05 |
| CO₂ | 0,000702835 | 1,92 | 0,001349444 |
| He | 7,02222E-06 | 6,28 | 4,40995E-05 |
| Ne | 8,34838E-06 | 3,01 | 2,51286E-05 |
| Ar | 2,73261E-05 | 1,98 | 5,41057E-05 |
| Kr | 5,03317E-05 | 1,92 | 9,66369E-05 |
| Xe | 8,80091E-05 | 1,85 | 0,000162817 |

## FIG.2

## FIG.3

# FIG.4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **Krooss B.M. ; Erdöl ; Kohle.** Diffusive Loss of Hydrocarbons through Cap Rocks. *Erdgas - Petrochemie/hydrocarbon Technology,* 1992, vol. 45, 387-396